(19) Europäisches Patentamt
European Patent Office
Office européen des brevets

(11) Veröffentlichungsnummer: **0 003 602**
**B1**

(12) # EUROPÄISCHE PATENTSCHRIFT

(45) Veröffentlichungstag der Patentschrift: **12.12.84**

(51) Int. Cl.³: **C 07 D 207/26**, A 61 K 31/40

(21) Anmeldenummer: **79100378.3**

(22) Anmeldetag: **09.02.79**

(54) **Pyrrolidin-Derivate zur Anwendung als therapeutische Wirkstoffe und diese enthaltende Arzneimittel.**

(30) Priorität: **10.02.78 CH 1404/78**

(43) Veröffentlichungstag der Anmeldung: **22.08.79 Patentblatt 79/17**

(45) Bekanntmachung des Hinweises auf die Patenterteilung: **12.12.84 Patentblatt 84/50**

(84) Benannte Vertragsstaaten: **BE CH DE FR GB IT LU NL SE**

(56) Entgegenhaltungen:
**FR - A - 2 294 698**

**CHEMICAL ABSTRACTS, vol. 87, nr. 4 25-7-1977 Columbus, Ohio, U.S.A. LABORATORIOS PHARMA. LAFARQUIM**
**CHEMICAL ABSTRACTS, vol. 87, nr. 7 15-8-1977 Columbus, Ohio, U.S.A. LABORATORIOS PHARMA. LAFARQUIM**

**Die Akte enthält technische Angaben, die nach dem Eingang der Anmeldung eingereicht wurden und die nicht in dieser Patentschrift enthalten sind.**

(73) Patentinhaber: **F. HOFFMANN-LA ROCHE & CO. Aktiengesellschaft, CH-4002 Basel (CH)**

(72) Erfinder: **Kyburz, Emilio, Dr., Unterer Rebbergweg 127, CH-4153 Reinach (CH)**
Erfinder: **Aschwanden, Werner, Grellingerstrasse 4, CH-4107 Ettingen (CH)**

(74) Vertreter: **Lederer, Franz, Dr. et al, Patentanwälte Lederer, Meyer Lucile-Grahn-Strasse 22, D-8000 München 80 (DE)**

**EP 0 003 602 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung betrifft Pyrrolidin-Derivate. Im speziellen betrifft sie 1-substituierte 2-Pyrrolidinone der allgemeinen Formel

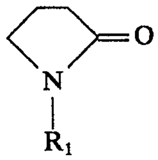

(I)

worin $R_1$ o-Methoxybenzoyl, m-Methoxybenzyl, p-Methoxybenzyl oder p-Fluorbenzyl bedeutet.

Diese Verbindungen sind bereits bekannt (Chem. Abstracts, 63, 16 256 e [1965], sowie FR-A-2 294 698), doch wurde überraschenderweise gefunden, daß sie bei geringer Toxizität interessante und therapeutisch verwertbare pharmakodynamische Eigenschaften aufweisen. In Tierversuchen konnte nämlich gezeigt werden, daß die Verbindungen der Formel I experimentell auf verschiedene Weise erzeugter cerebraler Insuffizienz entgegenzuwirken vermögen.

Gegenstand der vorliegenden Erfindung sind Verbindungen der allgemeinen Formel I zur Anwendung als therapeutische Wirkstoffe und Arzneimittel, enthaltend eine Verbindung der allgemeinen Formel I und ein pharmazeutisch inertes Excipiens. Die Verbindungen der allgemeinen Formel I können bei der Bekämpfung bzw. Verhütung von Krankheiten bzw. bei der Verbesserung der Gesundheit, insbesondere bei der Bekämpfung bzw. Verhütung der cerebralen Insuffizienz bzw. bei der Verbesserung der intellektuellen Leistungsfähigkeit verwendet werden.

Unter den Verbindungen der allgemeinen Formel I ist diejenige bevorzugt, worin $R_1$ p-Methoxybenzyl bedeutet, d. h. das 1-(p-Methoxybenzyl)-2-pyrrolidinon.

Die Fähigkeit von Verbindungen der Formel I, experimentell erzeugter cerebraler Insuffizienz entgegenzuwirken, konnte in den nachstehend beschriebenen Tierversuchen festgestellt werden:

A) Posthypercapnische »Avoidance«-Acquisition

Die Test-Apparatur ist eine »shuttle box« mit einer 10 cm hohen Hürde in der Mitte und elektrifizierbarem Gitterboden. In der schalldichten Kammer ist ein Lautsprecher montiert. Eine oder drei Stunden nach Verabreichung von Kontroll- oder Präparat-Injektionen werden unerfahrene Ratten (120—150 g; 10 pro Gruppe) für 12 Sekunden in reines $CO_2$-Milieu gebracht. Eine dritte Gruppe von 10 Ratten wird weder mit dem Präparat noch mit $CO_2$ behandelt. Drei Minuten nach $CO_2$-Behandlung müssen die Ratten aller drei Gruppen in der »shuttle box« in folgendem Programm einen bedingten und unbedingten Reflex erlernen: 10 Sek. Stille — 5 Sek. Ton (»avoidance response«) — 15 Sek. Ton + Fuß-Schock (»escape response«); sechsmal hintereinander. Für jeden der sechs Einzelversuche wird die Reaktionszeit (Zeit bis die Ratte über die Hürde springt) jeder Ratte gemessen und die statistische Signifikanz der Unterschiede zwischen den verschiedenen Gruppen mittels Rang-Test berechnet.

Als »aktiv« bezeichnet man diejenige Dosis eines Präparats, welche während der sechs Einzelversuche eine signifikante Wirkung zeigt; dabei müssen die mit dem Präparat und $CO_2$ behandelten Tiere signifikant besser lernen als die nur mit $CO_2$ behandelten Tiere und gleich gut wie die weder mit dem Präparat noch mit $CO_2$ behandelten Tiere.

In diesem Test zeigen die Verbindungen der Formel I bereits bei den folgenden Dosen eine signifikante Aktivität:

| $R_1$ | Erste signifikante wirksame Dosis |
|---|---|
| p-Methoxybenzyl | 10 mg/kg i.p. (nach 1 Std.) |
| | 50 mg/kg p.o. (nach 1 Std.) |
| p-Fluorbenzyl | 50 mg/kg i.p. (nach 1 Std.) |
| | 300 mg/kg p.o. (nach 1 Std.) |
| o-Methoxybenzoyl | 30 mg/kg i.p. (nach 1 Std.) |
| | 100 mg/kg p.o. (nach 1 Std.) |
| m-Methoxybenzyl | 50 mg/kg i.p. (nach 3 Std.) |

B) »Passive Avoidance«-Test mit Elektroschock-Amnesie

Die Testapparatur ist eine Skinnerbox mit elektrifizierbarem Gitterboden und einer grauen Viereckplattform in einer Ecke. Unerfahrene männliche Ratten, 100—200 g schwer, werden auf die graue Plattform gesetzt. Jedesmal wenn sie auf den Gitterboden hinuntersteigen, erhalten sie einen Elektroschock. Nach 3—5 Versuchen zeigen die Ratten eine sogenannte »passive avoidance response«, d. h. Weigerung von der Plattform herunterzusteigen. Unmittelbar nach Acquisition der Weigerung werden drei Gruppen zu je 20 Ratten gebildet. Eine Gruppe erhält einen Elektroschock (45 mA, 2 Sek.) durch die Ohren und eine i.p. NaCl-Injektion. Die zweite Gruppe erhält einen Elektroschock durch die Ohren und eine i.p. Injektion der Testsubstanz. Die dritte Gruppe erhält nur NaCl. Nach drei Stunden wird jede Ratte einmal auf die Plattform gesetzt und die Verweildauer (max. 60 Sek.) gemessen. Die signifikante Wirkung der Testsubstanz im Vergleich zu den beiden Kontrollgruppen wird mittels Rang-Test berechnet. Als »aktiv« bezeichnet man diejenige Dosis eines Präparates, welche eine signifikante Schutzwirkung gegen den Elektroschock zeigt (die mit aktiver Dosis eines Präparats und Elektroschock behandelten Tiere zeigen eine lange Verweildauer, ebenso die nicht mit Elektroschock behandelten Tiere, wogegen die mit NaCl und Elektroschock behandelten Tiere eine kurze Verweildauer zeigen).

In diesem Test beträgt die erste signifikant wirksame Dosis des 1-(p-Methoxybenzyl)-2-pyrrolidinons 50 mg/kg i.p.

C) Verzögerung des Haloperidol-induzierten »knock out«
in einem »continuous avoidance«-Programm mit Totenkopfaffen

Männliche, erwachsene Totenkopfaffen (Saimiri sciureus), 0,6 bis 1,2 kg schwer, einzeln gehalten, werden in einer 2-Tasten Skinnerbox auf folgendes »continuous avoidance«-Programm trainiert: »Avoidance-shock«-Intervall 40 Sek.; »shock-shock«-Intervall 20 Sek.; Fuß-Schock max. 5 Sek. Affen mit regelmäßiger Grundleistung erhalten Haloperidol 1,0 mg/kg p.o. zur Bestimmung der »knock-out«-Zeit (Blockierung von »avoidance« und »escape«). Affen mit stabilen »knock-out«-Zeiten werden für die Evaluation von Versuchspräparaten als potentielle cerebrale Insuffizienzverbesserer selektioniert. Die Präparate können zu verschiedenen Zeiten vor der Behandlung mit Haloperidol injiziert werden. Als »aktiv« bezeichnet man diejenige Dosis eines Präparates, welche bei Verabreichung vor der Behandlung mit Haloperidol eine signifikante Verzögerung des »knock out«-Zeitpunkts in einem 3-Stunden-Test bewirkt.

In diesem Test beträgt die erste signifikant wirksame Dosis des 1-(p-Methoxybenzyl)-2-pyrrolidinons 1 mg/kg i.p.

Bei der Ermittlung der akuten Toxizität der Verbindungen der Formel I an der Maus ergeben sich die folgenden Ergebnisse:

| $R_1$ | DL 50 |
|---|---|
| p-Methoxybenzyl | 2000—4000 mg/kg p.o. |
| p-Fluorbenzyl | 1000—2000 mg/kg p.o. |
| o-Methoxybenzoyl | 5000 mg/kg p.o. |
| m-Methoxybenzyl | 1250—2500 mg/kg p.o. |

Die Verbindungen der Formel I können als Heilmittel, z. B. in Form pharmazeutischer Präparate, Verwendung finden. Die pharmazeutischen Präparate können oral, z. B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z. B. in Form von Suppositorien, oder parenteral, z. B. in Form von Injektionslösungen, erfolgen.

Zur Herstellung von Tabletten, Lacktabletten, Dragées und Hartgelatinekapseln können die Verfahrensprodukte mit pharmazeutisch inerten, anorganischen oder organischen Excipientien verarbeitet werden. Als solche Excipientien kann man z. B. für Tabletten, Dragées und Hartgelatinekapseln Lactose, Maisstärke oder Derivate davon, Talk, Stearinsäure oder deren Salze usw. verwenden.

Für Weichgelatinekapseln eignen sich als Excipientien z. B. vegetabile Öle, Wachse, Fette, halbfeste und flüssige Polyole usw.

Zur Herstellung von Lösungen und Sirupen eignen sich als Excipientien z. B. Wasser, Polyole, Saccharose, Invertzucker, Glukose und dergleichen.

Für Injektionslösungen eignen sich als Excipientien z. B. Wasser, Alkohole, Polyole, Glyzerin, vegetabile Öle usw.

Für Suppositorien eignen sich als Excipientien z. B. natürliche oder gehärtete Öle, Wachse, Fette, halbflüssige oder flüssige Polyole und dergleichen.

Die pharmazeutischen Präparate können daneben noch Konservierungsmittel, Lösevermittler, Stabilisierungsmittel, Netzmittel, Emulgiermittel, Süßmittel, Färbemittel, Aromatisierungsmittel, Salze zur Veränderung des osmotischen Druckes, Puffer, Überzugsmittel oder Antioxidantien enthalten. Sie können auch noch andere therapeutisch wertvolle Stoffe enthalten.

Erfindungsgemäß kann man Verbindungen der allgemeinen Formel I bei der Bekämpfung bzw. Verhütung der cerebralen Insuffizienz bzw. bei der Verbesserung der intellektuellen Leistungsfähigkeit verwenden, beispielsweise bei cerebralen Insulten, in der Geriatrie, bei Alkoholismus usw. Die Dosierung kann innerhalb weiter Grenzen variieren und ist natürlich in jedem einzelnen Fall den individuellen Gegebenheiten anzupassen. Im allgemeinen dürfte bei oraler Verabreichung eine Tagesdosis von etwa 10 bis 2500 mg einer Verbindung der allgemeinen Formel I angemessen sein, wobei aber die soeben angegebene obere Grenze auch überschritten werden kann, wenn sich dies als angezeigt erweisen sollte.

In den nachfolgenden Beispielen, welche die vorliegende Erfindung illustrieren, ihren Umfang jedoch in keiner Weise beschränken sollen, werden pharmazeutische Präparate beschrieben, welche als Wirkstoff 1-(p-Methoxybenzyl)-2-pyrrolidinon enthalten.

## Beispiel 1

Gelatineweichkapseln, enthaltend 40 mg Wirkstoff pro Kapsel

| | pro Kapsel |
|---|---|
| Wirkstoff | 40 mg |
| Aus Kokosfett fraktioniertes Triglyceridgemisch | 150 mg |
| Kapselinhalt total | 190 mg |

## Beispiel 2

Injektionslösung, enthaltend 20 mg Wirkstoff pro ml

| | pro ml |
|---|---|
| Wirkstoff | 20,0 mg |
| Polyäthylenglycol 400 | 0,3 ml |
| Natriumchlorid | 2,7 mg |
| Wasser zu Injektionszwecken, ad | 1 ml |

## Beispiel 3

Flüssige orale Arzneiform (Emulsion), enthaltend 40 mg Wirkstoff pro 5 ml

| | pro 100 ml Emulsion |
|---|---|
| Wirkstoff | 0,8 g |
| Neutralöl | q.s. |
| Natriumcarboxymethylcellulose | 0,6 g |
| Polyoxyäthylen-stearat | q.s. |
| Glycerin rein | 0,2—2,0 g |
| Konservierungsmittel | q.s. |
| Geschmackskorrigens | q.s. |
| Wasser (entsalzt oder destilliert), ad | 100 ml |

## Beispiel 4

Rektale Arzneiform, enthaltend 40 mg Wirkstoff pro Suppositorium

| | pro Suppositorium |
|---|---|
| Wirkstoff | 40 mg |
| Suppositoriengrundmasse, ad | 2 g |

**Patentansprüche**

1. Pyrrolidin-Derivate der allgemeinen Formel

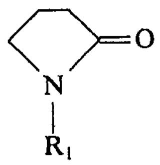

(I)

worin $R_1$ o-Methoxybenzoyl, m-Methoxybenzyl, p-Methoxybenzyl oder p-Fluorbenzyl bedeutet, zur Anwendung als therapeutische Wirkstoffe.

2. 1-(p-Methoxybenzyl)-2-pyrrolidinon zur Anwendung als therapeutischer Wirkstoff.

3. Pyrrolidin-Derivate der in Anspruch 1 angegebenen allgemeinen Formel I zur Anwendung als der cerebralen Insuffizienz entgegenwirkende bzw. die intellektuelle Leistungsfähigkeit verbessernde Wirkstoffe.

4. 1-(p-Methoxybenzyl)-2-pyrrolidinon zur Anwendung als der cerebralen Insuffizienz entgegenwirkender bzw. die intellektruelle Leistungsfähigkeit verbesernder Wirkstoff.

5. Arzneimittel, enthaltend ein Pyrrolidin-Derivat der in Anspruch 1 angegebenen allgemeinen Formel I und ein pharmazeutisch inertes Excipiens.

6. Der cerebralen Insuffizienz entgegenwirkendes bzw. die intellektuelle Leistungsfähigkeit verbesserndes Mittel, enthaltend ein Pyrrolidin-Derivat der in Anspruch 1 angegebenen allgemeinen Formel I und ein pharmazeutisch inertes Excipiens.

7. Arzneimittel, enthaltend 1-(p-Methoxybenzyl)-2-pyrrolidinon und ein pharmazeutisch inertes Excipiens.

8. Der cerebralen Insuffizienz entgegenwirkendes bzw. die intellektuelle Leistungsfähigkeit verbesserndes Mittel, enthaltend 1-(p-Methoxybenzyl)-2-pyrrolidinon und ein pharmazeutisch inertes Excipiens.

**Claims**

1. Pyrrolidine derivatives of the general formula

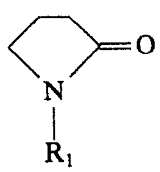

(I)

wherein $R_1$ signifies o-methoxybenzoyl, m-methoxybenzyl, p-methoxybenzyl or p-fluorobenzyl, for use as therapeutically active substances.

2. 1-(p-Methoxybenzyl)-2-pyrrolidinone for use as a therapeutically active substance.

3. Pyrrolidine derivatives of general formula I given in claim 1 for use as active substances counteracting cerebral insufficiency or improving intellectual capacity.

4. 1-(p-Methoxybenzyl)-2-pyrrolidinone for use as an active substance counteracting cerebral insufficiency or improving intellectual capacity.

5. A medicament containing a pyrrolidine derivative of general formula I given in claim 1 and a pharmaceutically inert excipient.

6. A medicament counteracting cerebral insufficiency or improving intellectual capacity, containing a pyrrolidine derivative of general formula I given in claim 1 and a pharmaceutically inert excipient.

7. A medicament containing 1-(p-methoxybenzyl)-2-pyrrolidinone and a pharmaceutically inert excipient.

8. A medicament counteracting cerebral insufficiency or improving intellectual capacity, containing 1-(p-methoxybenzyl)-2-pyrrolidinone and a pharmaceutically inert excipient.

**Revendications**

1. Dérivés pyrrolidiniques de formule générale

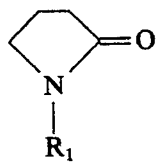

(I)

où $R_1$ représente un o-méthoxybenzoyle, m-méthoxybenzyle, p-méthoxybenzyle ou p-fluorobenzyle, pour leur application en tant que substances thérapeutiquement actives.

2. La 1-(p-méthoxybenzyl)-2-pyrrolidinone pour son application en tant que substance thérapeutiquement active.

3. Dérivés pyrrolidiniques de formule générale I de la revendication 1 pour leur application en tant que substances actives combattant l'insuffisance cérébrale ou améliorant la capacité intellectuelle.

4. La 1-(p-méthoxybenzyl)-2-pyrrolidinone pour son application en tant que substance active combattant l'insuffisance cérébrale ou améliorant la capacité intellectuelle.

5. Médicament contenant un dérivé pyrrolidinique de formule générale I selon la revendication 1 ainsi qu'un excipient pharmaceutiquement inerte.

6. Agent combattant l'insuffisance cérébrale ou améliorant la capacité intellectuelle, contenant un dérivé pyrrolidinique de formule générale I selon la revendication 1 ainsi qu'un excipient pharmaceutiquement inerte.

7. Médicament contenant la 1-(p-méthoxybenzyl)-2-pyrrolidinone ainsi qu'un excipient pharmaceutiquement inerte.

8. Agent combattant l'insuffisance cérébrale ou améliorant la capacité intellectuelle, contenant la 1-(p-méthoxybenzyl)-2-pyrrolidinone ainsi qu'un excipient pharmaceutiquement inerte.